# EUROPÄISCHE PATENTSCHRIFT

(11) **EP 0 925 111 B1**
(45) Veröffentlichungstag und Bekanntmachung des Hinweises auf die Patenterteilung: **11.07.2001**
(21) Anmeldenummer: 97944838.8
(22) Anmeldetag: 05.09.1997
(51) Int. Cl.: B01J 21/18

(54) **KATALYSATOR UND VERFAHREN ZU SEINER HERSTELLUNG**
CATALYST AND PROCESS FOR ITS MANUFACTURE
CATALYSEUR ET SON PROCEDE DE PRODUCTION

(30) Priorität: 06.09.1996 DE 19636269
(43) Veröffentlichungstag der Anmeldung: 30.06.1999
(73) Patentinhaber: Max-Planck-Gesellschaft zur Förderung der Wissenschaften e.V., 80539 München (DE)
(72) Erfinder: SCHLÖGL, Robert, D-14145 Berlin (DE); WOHLERS, Michael, D-65760 Eschborn (DE); BELZ, Thilo, D-14052 Berlin (DE); BRAUN, Thomas, D-12203 Berlin (DE)
(74) Vertreter: Huber, Bernhard, Dipl.-Chem.
(86) Internationale Anmeldenummer: EP9704842
(87) Internationale Veröffentlichungsnummer: WO9809725

(56) Entgegenhaltungen:
- WO-A-95/10481
- DE-A- 4 324 693
- GB-A- 2 217 701
- P.J.F.HARRIS: "Structure of non-graphitising carbons", INTERNATIONAL MATERIALS REVIEW, , 1997, Band 42, Nr. 5, Seiten 206 - 218

## Beschreibung

Die Erfindung betrifft einen Katalysator, der aus mindestens einem katalytisch aktivem Metall in niedervalentem Zustand auf einem im wesentlichen aus Kohlenstoff bestehenden Trägermaterial besteht, sowie in Verfahren zu dessen Herstellung und dessen Verwendung.

Für viele Anwendungen von katalytisch wirksamen Metallen auf Trägersystemen sind Trägersysteme auf Kohlenstoffbasis besonders vorteilhaft. Soweit dabei als Träger Material auf Graphitbasis verwendet werden, besteht ein Nachteil derselben jedoch in dessen ebenem Schichtenaufbau, der eine nur geringe attraktive Wechselwirkung mit den entsprechenden Metallen aufweist, so daß besonders unter Reaktionsbedingungen speziell bei erhöhter Temperatur das entsprechende Metall zur Agglomeration neigt. Dies bedingt unmittelbar eine Verringerung der katalytisch wirksamen Metalloberflächen, welche wiederum ein Absinken der katalytischen Aktivität des Katalysatorsystems nach sich zieht und damit insbesondere bei wertvollen Metallen eine ungenügende Ausnutzung der an sich vorhandenen Katalysekapazität zur Folge hat.

In der DE 43 24 693.1 der gleichen Erfinder wird bereits vorgeschlagen, diesen Nachteil dadurch zu beseitigen, daß man als Katalysatoren Metall-Fulleren-Interkalationsverbindungen verwendet. Diese haben den Vorteil, daß es sich um definierte Verbindungen handelt, die hohe Beständigkeit aufgrund guter Bindung der katalytisch aktiven Metalle mit exakter Reproduzierbarkeit verbinden. Dies liegt zu einem wesentlichen Anteil darin begründet, daß direkte kovalente Bindungen zwischen Kohlenstoffatomen des Fullerenmoleküls und einem Metallatom gebildet werden.

Aus der WO-A 95/10481 sind Katalysatoren bekannt, die aus einem Metall auf einem Kohlenstoffträgermaterials bestehen. Das Trägermaterial enthält ein Gebilde aus verkappten Kohlenstoffröhren ("Nanotubes") bzw. einen aus konzentrischen hohlen Polygonen bestehenden Kohlenstoff ("Nested Fullerenes"). Hier handelt es sich um ein kristallines Trägermaterial, welches ein Röntgendifraktionsbild aufweist, welches identisch ist mit dem von Graphit.

Ein Nachteil der Metall-Fulleren-Interkalationsverbindungen als Katalysatoren liegt im hohen Preis des Trägermaterials. Zudem wäre eine nochmalige Steigerung der katalytischen Wirksamkeit, jeweils bezogen auf den Gehalt an katalytisch aktivem Metall bzw. Metallverbindung erwünscht durch Verbesserung der Zugänglichkeit des Metalls für die Komponenten der katalysierten Reaktion. Der Erfindung liegt daher die Aufgabe zugrunde, die vorstehenden Nachteile bei einem Katalysator mit einem Träger auf Kohlenstoffbasis zu beseitigen.

Gelöst wird diese Aufgabe erfindungsgemäß durch einen Katalysator, bestehend aus mindestens einem katalytisch aktiven Metall in niedervalentem Zustand auf einem im wesentlichen aus Kohlenstoff bestehenden Trägermaterial, der dadurch gekennzeichnet ist, daß das Trägermaterial aus Kohlenstoff besteht, der in amorphem Zustand mit gekrümmten Oberflächen der molekularen Ebenen, die nicht-sechsgliedrige Kohlenstoff-Ringe enthalten, vorliegt und das katalytisch aktive Metall kovalent an das Kohlenstoff-Trägermaterial gebunden ist.

Der Erfindung liegt die überraschende Feststellung zugrunde, daß im wesentlichen aus Kohlenstoff bestehende Trägermaterialien, die amorph vorliegen und gekrümmte Oberflächen der molekularen Ebenen aufweisen, die nicht nur sechsgliedrige sondern auch nicht-sechsgliedrige Kohlenstoffringe enthalten, sich als Trägermaterial für katalytisch aktive Metalle eignen und hierbei besonders gute Eigenschaften, die zu überlegenen Katalysatoren führen, bewirken. Es wird angenommen, daß dies auf dem Vorhandensein gekrümmter sp²-hybridisierter Kohlenstoffschichten beruht. Bei dieser Schichtenkrümmung handelt es sich nicht um ein einfaches "Rollen" oder "Biegen" von ansonsten intakten Graphitschichten, sondern die Krümmung resultiert aus dem Einbau von nicht-sechsgliedrigen Ringen in die sp²-hybridisierten Kohlenstoffschichten. Die so erzeugte Krümmung der Kohlenstoffschichten ist mit signifikanten Abweichungen von der geometrischen und elektronischen Struktur einer planaren, graphitischen sp²-hybridisierten Kohlenstoffschicht verbunden. Neben einem erheblichen Zugewinn an potentieller Energie (Spannungsenergie) erfolgt innerhalb dieser gekrümmten Bereiche keine vollständige Delokalisation der vorhandenen π-Elektronen, so daß die gekrümmten -Kohlenstoffschichten gewissermaßen als konjugierte Doppelbindungssysteme aufgefaßt werden können.

Die in den erfindungsgemäßen Katalysatoren enthaltenen Kohlenstoffträgermaterialien sind daher in Analogie zu elektronenarmen Olefinen in der Lage, chemische Bindungen zu Übergangsmetallen in niedrigen formalen Oxidationsstufen auszubilden. Es handelt sich hierbei um direkte chemische Bindungen zwischen Metall und Kohlenstoffträger, die nicht wie bei den bekannten konventionellen Kohlenstoffträgern, wie z.B. Aktivkohle, die Metallatome hauptsächlich über Wechselwirkungen mit terminalen Heteroatomfunktionalitäten, hauptsächlich Sauerstoff, verankern.

Die in den erfindungsgemäßen Katalysatoren enthaltenen Kohlenstoffmaterialien bestehen aus einem Geflecht von 6er-Ringen, in die weitere Ringe, insbesondere solche aus fünf Kohlenstoffatomen, eingebaut sind. Diese gekrümmten Oberflächen können konkav oder konvex ausgebildet sein. Diese für das Trägermaterial im Rahmen der Erfindung essentielle Struktur kann durch physikalische Methoden, insbesondere Röntgenstrahlen-Absorptionsspektroskopie (XAS) bestimmt werden, wie in H. Werner et al., Chem. Phys. Letters 194 (1992), 62-66 beschrieben. Weiterhin lassen sich diese gekrümmten Bereiche durch ihre spezielle chemische Reaktivität charakterisieren, entsprechende Reaktionsprodukte wurden infrarotspektroskopisch nach partieller Oxidation dieser Kohlenstoffmaterialien charakterisiert (M. Wohlers, A. Bauer, R. Schlögl, Microchim. Acta, eingereicht 1995, im Druck). Ein Beispiel für die im Rahmen der Erfindung geeigneten Materialien ist der sogenannte "Krätschmerruß". Hierbei handelt es sich um ein Produkt, das bei der Fullerenherstellung nach Krätschmer nach Abtrennung der Fullerene übrig bleibt. Wie weiter unten noch näher beschrieben, kann das Trägermaterial auch nach anderen Methoden, außer der von Krätschmer beschriebenen, erhalten werden.

Die Fähigkeit zur kovalenten Bindung im Rahmen der erfindungsgemäßen Katalysatoren weisen Metallpartikel auf, die ca. 10 bis 1000 Atome enthalten. Bei größeren Metallpartikeln sinkt die Bindefestigkeit ab und erreicht bei zunehmender Größe der Metallpartikel schließlich Werte, die, auf die Größe der Metallpartikel bezogen, nicht mehr wesentlich über den Bindungsfestigkeiten konventioneller Kohlenstoffträger liegen.

Von Fulleren unterscheiden sich die im erfindungsgemäßen Katalysator enthaltenen Trägermaterialien dadurch, daß die in den gebogenen Strukturelementen vorhandenen reaktiven Doppelbindungen integraler Bestandteil des nicht-molekularen Kohlenstoffmaterials sind, während Fullerene aus definierten Einzelmolekülen bestehen. So gehört beispielsweise zu den erfindungsgemäß geeigneten Trägermaterialien der sogenannte "Zwiebelkohlenstoff". Er weist zwiebelartige Strukturen auf mit Durchmessern von 100 Å oder mehr, welche fest in das umgebende Kohlenstoffmaterial eingebetten sind, während die Fullerene einen Durchmesser von etwa 10 Angström aufweisen und molekular sind.

Zur Herstellung der im erfindungsgemäßen Katalysator enthaltenen Trägermaterialien lassen sich neben dem schon erwähnten Verfahren von Krätschmer und anschließender Abtrennung der Fullerene daraus, insbesondere die Hochfrequenzverdampfung von Graphit, die Laserverdampfung von Graphit sowie die Pyrolyse von Kohlenwasserstoffen unter geeigneten Bedingungen anführen. Insbesondere läßt sich das Trägermaterial durch Verdampfung von reinem Graphit in inerter Atmosphäre und Abschreckung des Dampfes und nachfolgende Lösungsmittelextraktion etwa gebildeter Fullerene herstellen.

Die überlegenen katalytischen Eigenschaften der erfindungsgemäßen Katalysatoren werden darauf zurückgeführt, daß alles katalytisch aktive Metall an der Oberfläche fixiert vorliegt und daher für die Komponenten der zu katalysierenden Reaktionen sehr leicht zugänglich ist, während bei den Übergangsmetall-Fullerenverbindungen ein nicht zu vernachlässigender Teil des katalytisch aktiven Metalls im Inneren der jeweiligen makroskopischen Partikel der Übergangsmetall-Fullerenverbindungen vorliegt und daher für die Komponenten der zu katalysierenden Reaktion nicht mehr zugänglich ist. Die überlegene Beständigkeit gegenüber auf Graphitbasis aufgebauten Katalysatoren wird der kovalenten Bindung des Metalls zugeschrieben.

Unter niedervalenten Metallen werden im Rahmen der vorliegenden Erfindung die nullwertigen, eins- und zweiwertigen Zustände verstanden. Die Metalle werden bevorzugt in nullwertiger Form eingesetzt mit den bekannten stabilisierenden Liganden wie z.B. Carbonyle, Isonitrile, Phosphine, Phosphite, Al-kene, Polyalkene, Heteroalkene, Alkine, sowie cyclisch konjugierte Systeme wie z.B. Benzol oder Cyclopentadienyl-Anionen. Beispiele für geeignete nullwertige katalytisch aktive Metallkomplexe sind z.B. Trirutheniumdodecacarbonyl, Platindibenzylidenaceton, Palladiumdibenzylidenaceton, Palladiumtetrakistriethylphosphin, Nickeltetracarbonyl, Eisenpentacarbonyl, Di-Eisennonacarbonyl, Trieisendodecacarbonyl u.ä. Diese Verbindungen sind teilweise handelsüblich oder lassen sich nach bekannten Verfahren, wie sie beispielsweise im Handbuch der präparativen anorganischen Chemie, Band 3, F. Encke Verlag, Stuttgart, beschrieben sind, erhalten. Geeignete ein- und zweiwertige Verbindungen sind solche, die unter Reaktionsbedingungen, gegebenenfalls unter Zusatz eines als Reduktionsmittel wirkenden Reagenzes wie beispielsweise molekularer Wasserstoff, Kohlenmonoxid oder Natriumborhydrid, in den nullwertigen Zustand reduziert werden können. Als katalytisch aktive Metalle werden vorzugsweise solchen aus den Gruppen Ib, VIIb, VIIIb des periodischen Systems, den niedervalente Verbindungen bildenden SE-Metalle, Titan und Vanadium bevorzugt. Besonders bevorzugte Metalle für die erfindungsgemäßen Katalysatoren sind insbesondere Platin, Ruthenium, Palladium und Eisen. Weitere geeignete Metalle sind z.B. Nickel, Kobalt, Mangan, Osmium, Iridium und Rhenium, desgleichen Titan und Vanadium soweit sie in den niedervalenten Zustand gebracht werden können.

Ein besonders interessanter Aspekt der Erfindung liegt in den erzielbaren qualitativen Änderungen der katalytischen Eigenschaften der gebundenen Metalle. Es konnte gezeigt werden, daß die in den erfindungsgemäß beschriebenen Systemen enthaltenen Metallpartikel sich in bezug auf ihre strukturellen Eigenschaften deutlich von denen vergleichbarer Metall/Graphit-Systeme unterscheiden. Diese strukturellen Unterschiede werden auf qualitativ unterschiedliche attraktive Wechselwirkungen der Metallpartikel mit den jeweiligen Kohlenstoffträgern zurückgeführt. Die beobachteten strukturellen Unterschiede der Metallpartikel sind allerdings nicht ausschließlich geometrischer Natur, sondern es wird angenommen, daß auch die elektronische Struktur der Metallpartikel Unterschiede aufweist. Letztendlich führt dies dazu, daß die für die heterogenkatalytischen Umsetzungen entscheidenden aktiven Zentren an der Metalloberfläche unterschiedliche Eigenschaften aufweisen. So wird typischerweise bei der Kohlenmonoxidhydrierung eine Selektivitätsverschiebung zu höhermolekularen Produkten, die in erster Linie bei diesem Verfahren angestrebt werden, bei Einsatz der erfindungsgemäßen Katalysatoren erzielt.

Gegenüber Katalysatoren mit Graphit als Träger, besteht ein genereller Vorteil der erfindungsgemäßen Katalysatoren darin, daß sie noch bei höheren Temperaturen stabil sind. Hinzu kommt, daß sie in vielen Fällen auch quantitativ unter sonst vergleichbaren Bedingungen bessere und in anderen Fällen auch qualitativ andersartige katalytische Eigenschaften zeigen.

Ein weiterer Gegenstand der Erfindung ist ein Verfahren zur Herstellung der neuen Katalysatoren. Das erfindungsgemäße Verfahren zur Herstellung der neuen Katalysatoren besteht darin, daß man Graphit im elektrischen Lichtbogen zwischen mindestens zwei Graphitelektroden in einer Vakuumapparatur unter nichtoxidierender Atmosphäre verdampft, wobei man entweder
a) in einer Vakuumapparatur mit gekühlten Wänden mit Gleich- oder Wechselstrom unter einem Druck von 100 Pa oder weniger arbeitet und das an den gekühlten Wänden abgeschiedene Produkt gewinnt, oder
b) mit Gleichstrom bei einem Druck von 1 Pa bis 100 kPa und Bogenlängen von 0,1 bis 20 mm arbeitet und das auf der mit dem Minuspol der Stromversorgung verbundenen Elektrode aufgewachsene Produkt gewinnt und
c) mit Wechselstrom bei einem Druck von 1 Pa bis 100 Pa und Bogenlängen von 0,1 bis 20 mm arbeitet und das auf den Kohlenstoffelektroden aufgewachsene Produkt gewinnt und
d) das Produkt von a), b) oder c) mit einer thermolabilen niederwertigen Verbindung oder Komplex eines katalytisch aktiven Metalls umsetzt.

Der verwendete Graphit sollte möglichst rein sein. Als Atmosphäre wird Edelgas bevorzugt, insbesondere Helium, Argon oder eine Mischung von Helium und Argon. Andere brauchbare Gase sind z.B. Wasserstoff, Stickstoff oder Ammoniak.

Wird die Herstellung des Trägermaterials gemäß der oben beschriebenen Variante a) durchgeführt, so wird zweckmäßig die Kühlung der Wände der Vakuumapparatur mit Wasser vorgenommen. Andere Kühlungsmethoden oder Kühlmittel können jedoch in gleicher Weise angewendet werden. Ferner wird bei der Herstellung des Trägermaterials vorzugsweise mit zwei Graphitelektroden gearbeitet, da handelsüblich erhältliche Vorrichtungen in der Regel hierfür eingerichtet sind. Im Rahmen der Erfindung können jedoch auch mehr als zwei Graphitelektroden eingesetzt werden bei entsprechend abgewandelter Lichtbogenapparatur.

Wie oben bereits erwähnt, kann als Trägermaterial auch ein nach dem Verfahren von Krätschmer (W. Krätschmer et al., Chemical Physics Letters, Vol. 170 (1990), Seite 167 bis 170) hergestelltes und danach durch Extraktion von gebildeten Fullerenen befreites Material eingesetzt werden.

Die Umsetzung des Trägermaterials mit der Metallverbindung wird vorzugsweise in einer Suspension des Trägermaterialkohlenstoffs in einem Lösungsmittel für die Metallverbindung unter Luftausschluß durchgeführt. Dabei wird zweckmäßig bei erhöhter Temperatur gearbeitet, vorzugsweise bei Rückflußtemperatur des Lösungsmittels. Die Umsetzung unter diesen Bedingungen wird im allgemeinen zwischen 1 und 50 Stunden, vorzugsweise 15 bis 30 Stunden durchgeführt. Sollten Fullerene im Trägermaterial enthalten sein, werden sie vorher entfernt, vorzugsweise durch Extraktion mit einem der hierfür geeigneten organischen Lösungsmittel.

Der allgemeine Temperaturbereich für die Umsetzung des Trägerma-terials mit dem Metall liegt zwischen dem Erstarrungspunkt des Lösungsmittels und seinem Siedepunkt. Durch Druckanwendung kann die Siedetemperatur des Lösungsmittels in bekannter Weise erhöht werden, wobei Drücke bis 100 MPa, vorzugsweise bis etwa 10 MPa zur Anwendung kommen können.

Geeignete Lösungsmittel sind Aromaten, halogenierte Aromaten, chlororganische Verbindungen und Heterocyclen wie Benzol, Toluol, Xylol, Ethylbenzol, Chlorbenzol, Dichlorbenzol, Tetrachlorkohlenstoff, Chloroform, Dichlormethan und Tetrahydrofuran.

Erfindungsgemäße Katalysatoren eignen sich generell für Umsetzungen, die unter Übergangsmetallkatalyse ablaufen. Bevorzugt wird der Einsatz zur Kohlenmonoxidhydrierung. Weitere Umsetzungen, für die sich die erfindungsgemäßen Katalysatoren besonders eignen, sind z.B. Flüssigphasenhydrierungen organischer Moleküle, welche vorzugsweise im Temperaturbereich zwischen Erstarrungspunkt der zu hydrierenden Verbindung oder des verwendeten Lösungsmittels und 150°C durchgeführt werden.

Derartige Flüssigphasenhydrierungen eignen sich beispielsweise für die Hydrierung von Olefinen, Ketonen, aromatischen Nitroverbindungen und Substanzen mit vergleichbarer Reaktivität. Diese Hydrierungen können sowohl in Substanz (falls die zu reduzierende Verbindung unter den Hydrierungsbedingungen flüssig ist) als auch in Lösung durchgeführt werden. Hierfür geeignete Lösungsmittel sind z.B. Tetrahydrofuran, Methylenchlorid oder Toluol.

Das folgende Beispiel erläutert die Erfindung anhand der Kohlenmonoxidhydrierung mit einem Ruthenium-haltigen Katalysator gemäß Erfindung in Verbindung mit der Zeichnung weiter. In dieser zeigt

### Figur 1

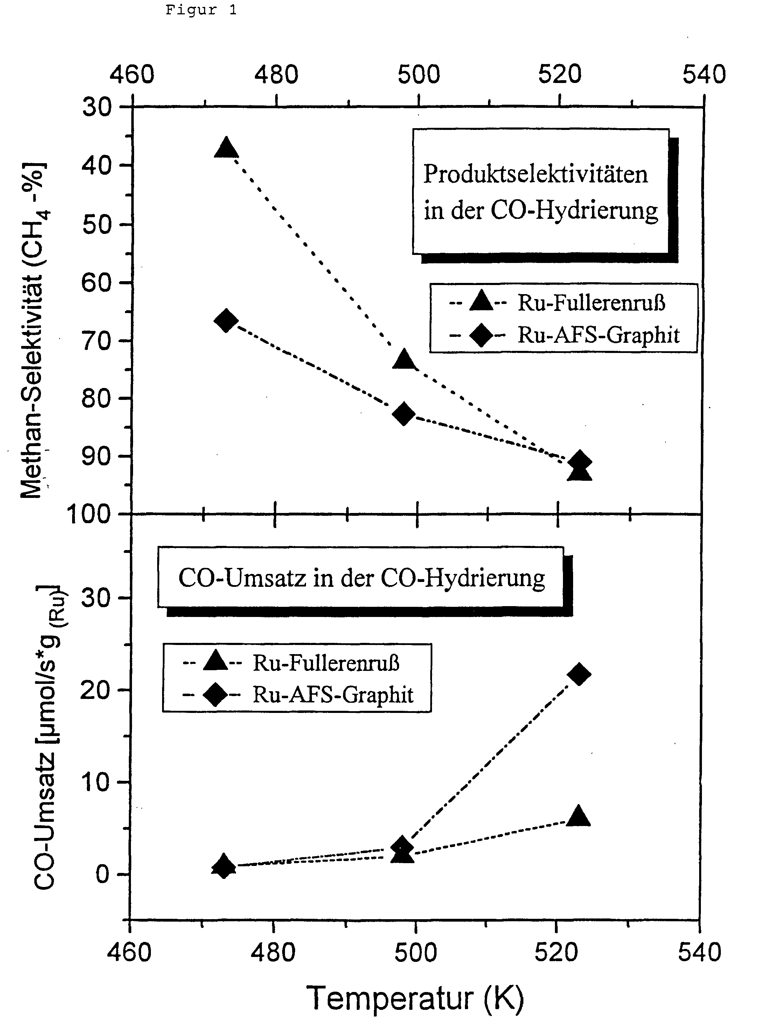

CO-Umsatz und Produktselektivität von Ruthenium-haltigem erfi-ndungsgemäßem Katalysator und Ruthenium-AFS-Graphit. Im unteren Teil der Abbildung wird der gaschromatographisch ermittelte CO-Umsatz der Katalysatoren bei verschiedenen Temperaturen verglichen; dieser ist ein Maß für die heterogenkatalytische Aktivität der Systeme. Im oberen Teil wird analog die Methan-Selektivität der beiden Systeme verglichen. Die Methan-Selektivität ist gegeben durch den prozentualen Anteil von Methan in dem resultierenden Kohlenwasserstoffgemisch, welches u.a. Ethan, Ethen und weitere Aliphate bis hin zu Oktan enthält. In diesem Teil von Fig. 1 wird deutlich, daß der erfindungsgemäße Katalysator bei niedrigen Temperaturen einen höheren Anteil höherer Kohlenwasserstoffe produziert als der korrespondierende Ru-Graphit Katalysator.
Fig. 1 zeigt damit die bessere Produktselektivität der erfindungsgemäßen Katalysatoren.

### Figur 2

Hier wird analog zu Fig. 1 ein Vergleich der katalytischen Aktivitäten und der Produktselektivitäten von Ru-Kathodenpils gemäß Erfindung und Ru-AFS-Graphit angestellt. Man erkennt aus dieser Abbildung, daß der Kathodenpils bei nahezu identischer Produktselektivität speziell bei hohen Temperaturen eine signifikant höhere katalytische Aktivität aufweist als das Referenzsystem auf Graphitbasis. Die Verringerung der katalytischen Aktivität des Graphitsystems konnte auf eine Agglomeration der Metallpartikel zurückgeführt werden, die eine Verringerung der katalytisch aktiven Metalloberfläche zur Folge hat.

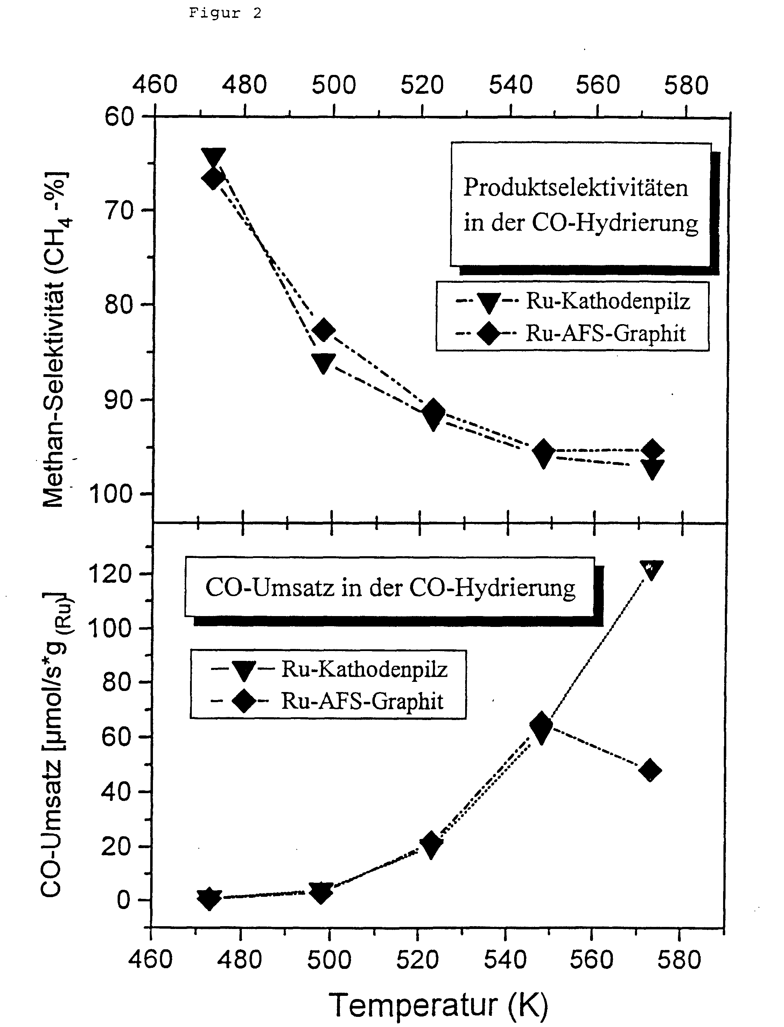

Fig. 2 zeigt damit eine bessere thermische Stabilität unter Reaktionsbedingungen der Katalysatoren gemäß Erfindung.

### Beispiel

a. Herstellung des Rutheniumkatalysators
   1. Graphit wurde im elektrischen Lichtbogen zwischen zwei Graphitelektroden, die mit Wechselstrom betrieben wurden, in einer wassergekühlten Vakuumapparatur unter Heliumatmosphäre bei einem Druck von 100 Pa verdampft.. Das gewünschte Produkt sammelt sich an den wassergekühlten Wänden der Apparatur. Es wird nach Beendigung der Verdampfung und Abkühlung der Apparatur entnommen und in Toluol suspendiert. Dieser Suspension wurde Trirutheniumdodecacarbonyl Ru₃(CO)₁₂ bei Raumtemperatur zugesetzt und gelöst. Die erhaltene Suspension wurde langsam zum Sieden erhitzt und 1 Tag am Rückfluß gehalten. Dann wurde der unlösliche Katalysator vom Lösungsmittel abgetrennt, mit dem Lösungsmittel gewaschen und im Vakuum bei Raumtemperatur getrocknet. Der erhaltene Katalysator wurde ohne weitere Vorbehandlung in der Katalyse eingesetzt.
   2. Graphit wurde im elektrischen Lichtbogen zwischen zwei Graphitelektroden, die mit Gleichstrom betrieben wurden, in einer Vakuumapparatur unter Heliumatmosphäre bei einem Druck von 0,6 kPa und einer Bogenlänge von 1 mm verdampft. Das auf der mit dem Minuspol der Stromversorgung verbundenen Graphitelektrode aufgewachsene Produkt wurde wie unter 1. beschrieben gewonnen, vermahlen und zum Rutheniumkatalysator umgesetzt.
b. Kohlenmonoxidhydrierung

Die heterogene Hydrierung von Kohlenmonoxid wurde in einem Festbettdurchflußreaktor bei Atmosphärendruck im Temperaturbereich von 200 bis 300°C durchgeführt. Als Kontakt wurde eine 2 cm hohe Schüttung, bestehend aus 100 mg nach a.1.) erhaltenem Katalysator und der entsprechenden Menge inerter Glaskugeln verwendet. Die nachgereinigten Eduktgase wurden in einem H₂:CO-Verhältnis von 3:1 bei einem Gesamtdurchfluß von 20 ml/min eingesetzt. Die Produktgase wurden mit einem an die Syntheseapparatur-gekoppelten Gaschromatographen bestimmt.

Parallel hierzu wurden unter sonst gleichen Bedingungen Rutheniumkatalysatoren verwendet, die an Graphit gebunden vorlagen. Die erzielten Ergebnisse sind in der beigefügten Zeichnung dargestellt.

## Patentansprüche

1. Katalysator, bestehend aus mindestens einem katalytisch aktiven Metall in nullwertigem, ein- oder zweiwertigem Zustand auf einem im wesentlichen aus Kohlenstoff bestehenden Trägermaterial,
**dadurch gekennzeichnet**,
daß das Trägermaterial aus Kohlenstoff besteht, der in amorphem Zustand mit gekrümmten Oberflächen der molekularen Ebenen die nichtsechsgliedrige Kohlenstoff-Ringe enthalten, vorliegt und das katalytisch aktive Metall kovalent an das Kohlenstoff-Trägermaterial gebunden ist.

2. Katalysator nach Anspruch 1,
**dadurch gekennzeichnet**,
daß das Metall in Form von Atomen, Clustern oder Partikeln mit 10 bis 1000 Atome gebunden ist.

3. Katalysator nach Anspruch 1 oder 2,
**dadurch gekennzeichnet**,
daß das Trägermaterial aus dem nach Abtrennung der Fullerene erhaltenen Rückstand von Fullerenruß besteht.

4. Katalysator nach Anspruch 3,
**dadurch gekennzeichnet**,
daß das Trägermaterial aus dem nach Lösungsmittelextraktion der Fullerene erhaltenen Rückstand der Fullerenherstellung durch Verdampfung von reinem Graphit in inerter Atmosphäre und Abschreckung des Dampfes besteht.

5. Katalysator nach einem der vorhergehenden Ansprüche,
**dadurch gekennzeichnet**,
daß das katalytisch aktive Metall ausgewählt ist aus den Gruppen Ib, VIIb, VIIIb, den niedervalente Verbindungen bildenden Seltenerdmetallen, Titan und Vanadium.

6. Verfahren zur Herstellung eines Katalysators nach einem der Ansprüche 1 bis 5, **dadurch gekennzeichnet**,
daß man Graphit im elektrischen Lichtbogen zwischen mindestens zwei Graphitelektroden in einer Vakuumapparatur unter nichtoxidierender Atmosphäre verdampft, wobei man
a) in einer Vakuumapparatur mit gekühlten Wänden mit Gleich- oder Wechselstrom unter einem Druck von 100 Pa oder weniger arbeitet und das an den gekühlten Wänden abgeschiedene Produkt gewinnt, oder
b) mit Gleichstrom bei einem Druck von 1 Pa bis 100 kPa und Bogenlängen von 0,1 bis 20 mm arbeitet und daß auf der mit dem Minuspol der Stromversorgung verbundenen Elektrode aufgewachsene Produkt gewinnt und
c) mit Wechselstrom bei einem Druck von 1 Pa bis 100 Pa und Bogenlängen von 0,1 bis 20 mm arbeitet und das auf den Kohlenstoffelektroden aufgewachsene Produkt gewinnt und
d) das Produkt von a), b) oder c) mit einer thermolabilen niederwertigen Verbindung oder Komplex eines katalytisch aktiven Metalls umsetzt.

7. Verfahren nach Anspruch 6,
**dadurch gekennzeichnet**,
daß man die Umsetzung mit der Metallverbindung in Stufe d) in einer Suspension des Trägermaterialkohlenstoffs in einem Lösungsmittel für die Metallverbindung unter Luftausschluß durchführt.

8. Verfahren nach Anspruch 7,
**dadurch gekennzeichnet**,
daß man die Umsetzung bei Rückflußtemperatur des Lösungsmittels durchführt.

9. Verfahren nach einem der Ansprüche 6 bis 8,
**dadurch gekennzeichnet**,
daß man die Wände der Vakuumapparatur in Stufe a) mit Wasser kühlt.

10. Verfahren nach einem der Ansprüche 6 bis 9,
**dadurch gekennzeichnet**,
daß man unter Helium- oder Argonatmosphäre arbeitet.

11. Verwendung eines Katalysators nach einem der Ansprüche 1 bis 5 für die Kohlenmonoxidhydrierung.

12. Verwendung eines Katalysators nach einem der Ansprüche 1 bis 5 für die Flüssigphasenhydrierung organischer Moleküle.

## Claims

1. A catalyst which consists of at least one catalytically active metal in an avalent, monovalent or divalent state on an essentially carbon substrate,
**characterized in that**
the substrate consists of amorphous carbon with molecular planes that have curved surfaces and contain non-six-membered carbon rings, and the catalytically active metal is bound covalently to the carbon substrate.

2. The catalyst of claim 1,
**characterized in that**
the metal is bound in the form of atoms, clusters or particles with 10 to 1 000 atoms.

3. The catalyst of claim 1 or 2,
**characterized in that**
the substrate consists of the fullerene soot residue obtained after the fullerenes have been separated off.

4. The catalyst of claim 3,
**characterized in that**
the substrate consists of the residue obtained when fullerenes are prepared by vaporizing pure graphite in an inert atmosphere and rapidly cooling the vapor, and the fullerenes are then removed by solvent extraction.

5. The catalyst according to one of the preceding claims,
**characterized in that**
the catalytically active metal is selected from the groups lb, Vllb, Vlllb, rare-earth metals that form low-valence compounds, or titanium and vanadium.

6. A method of producing a catalyst as claimed in one of claims 1 to 5,
**characterized in that**
graphite is vaporized in a non-oxidizing atmosphere by means of an electric arc struck between at least two graphite electrodes in a vacuum apparatus, during which process one
a) works with a.c. or d.c. under a pressure of 100 Pa or less in a vacuum apparatus the walls of which are cooled, the product being deposited on the cooled walls, or
b) with d.c. under a pressure of 1 to 100 kPa and arc lengths of 0.1 to 20 mm, the product accumulating on the electrode connected to the negative pole of the the power supply, or
c) with a.c. under a pressure of 1 to 100 Pa and arc lengths of 0.1 to 20 mm, the product accumulating on the carbon electrodes, and then
d) reacts the product of a), b) or c) with a thermolabile, low-valence compound or complex of a catalytically active metal.

7. The method of claim 6,
**characterized in that**
the reaction with the metal compound as per step d) is performed in the absence of air in a suspension of the carbon substrate in a solvent in which the metal compound is soluble.

8. The method of claim 7,
**characterized in that**
the reaction is carried out at the reflux temperature of the solvent.

9. The method according to one of claims 6 to 8,
**characterized in that**
the walls of the vacuum apparatus as per alternative a) are cooled with water.

10. The method according to one of claims 6 to 9,
**characterized in that**
a working atmosphere of helium or argon is used.

11. Use of a catalyst according to one of claims 1 to 5 for the hydrogenation of carbon monoxide.

12. Use of a catalyst according to one of claims 1 to 5 for the liquid-phase hydrogenation of organic molecules.

## Revendications

1. Catalyseur, consistant au moins en un métal catalytiquement actif à l'état nonvalent, monovalent ou bivalent sur un matériau porteur constitué essentiellement par du carbone,
caractérisé en ce que,
le matériau porteur consiste en carbone qui se présente à l'état amorphe avec les surfaces courbées des plans moléculaires qui contiennent les cycles de carbone n'ayant pas six chaînons et le métal catalytiquement actif est lié au matériau porteur de carbone.

2. Catalyseur selon la revendication 1,
caractérisé en ce que,
le métal est lié sous forme d'atomes, d'agglomérats ou de particules avec 10 jusqu'à 1000 atomes.

3. Catalyseur selon la revendication 1 ou 2,
caractérisé en ce que,
le matériau porteur consiste en résidus de suie de fullerènes obtenus après la séparation des fullerènes.

4. Catalyseur selon la revendication 3,
caractérisé en ce que,
le matériau porteur consiste en résidus obtenus par extraction par solvant des fullerènes dans la préparation de fullerènes par évaporation du graphite pur en atmosphère inerte et refroidissement brusque de la vapeur.

5. Catalyseur selon l'une des revendications précédentes,
caractérisé en ce que,
le métal catalytiquement actif est choisi parmi les groupes Ib, VIIb, VIIIb, les métaux des terres rares formant des composés à faible valence, le titane et le vanadium.

6. Procédé pour la préparation d'un catalyseur selon l'une des revendications 1 à 5,
caractérisé en ce que,
l'on vaporise le graphite sous arc électrique entre au moins deux électrodes de graphite dans un appareil sous vide sous atmosphère non oxydante,
a) en travaillant dans un appareil sous vide comportant des parois refroidies avec un courant continu ou alternatif sous une pression de 100 Pa ou moins et l'on obtient le produit précipité sur les parois refroidies, ou
b) en travaillant avec un courant continu sous une pression de 1 Pa à 100 kPa et des longueurs d'arc de 0,1 à 20 mm et en ce que l'on obtient le produit déposé sur l'électrode raccordée au pôle moins de l'alimentation électrique et
c) en travaillant avec un courant alternatif sous une pression de 1 Pa jusqu'à 100 Pa et des longueurs d'arc de 0,1 à 20 mm et en ce que l'on obtient le produit déposé sur les électrodes de carbone et
d) en faisant réagir le produit de a),b) ou c) avec un complexe ou composé thermolabile à faible valence d'un métal catalytiquement actif.

7. Procédé selon la revendication 6,
caractérisé en ce que,
l'on conduit la réaction avec le composé métallique à l'étape d) dans une suspension du carbone du matériau porteur dans un solvant pour le composé métallique sous exclusion d'air.

8. Procédé selon la revendication 7,
caractérisé en ce que,
l'on conduit la réaction à la température de reflux du solvant.

9. Procédé selon l'une des revendications 6 à 8,
caractérisé en ce que,
l'on refroidit les parois de l'appareil sous vide à l'étape a) avec de l'eau.

10. Procédé selon l'une des revendications 6 à 9,
caractérisé en ce que,
l'on travaille sous atmosphère d'hélium ou d'argon.

11. Utilisation d'un catalyseur selon l'une des revendications 1 à 5 pour l'hydratation de monoxyde de carbone.

12. Utilisation d'un catalyseur selon l'une des revendications 1 à 5 pour l'hydratation en phase liquide de molécules organiques.
